# EUROPEAN PATENT APPLICATION

(11) **EP 1 632 260 A1**
(43) Date of publication of application: **08.03.2006**
(21) Application number: 04733171.5
(22) Date of filing: 14.05.2004
(51) Int. Cl.: A61M 15/00

(54) **INHALATION TYPE MEDICATION APPARATUS**

(30) Priority: 11.06.2003 JP 2003166802
(71) Applicant: Hitachi, Ltd., Tokyo 100-8280 (JP); DOTT LIMITED COMPANY, Yokohama-shi, Kanagawa 224-0051 (JP)
(72) Inventor: OHKI, Hisatomo c/o Automotive Systems,Hitachi,Ltd., Gunma, 372-0023 (JP); NAKAMURA,Shigemic/o Automot.Systems,Hitachi,Ltd., Gunma, 372-0023 (JP); ISHIZEKI, Kazunori C/O Automot.Systems,Hitachi Ltd, Gunma, 372-0023 (JP); YANAGAWA, Akira, . (JP)
(74) Representative: Grünecker, Kinkeldey, Stockmair & Schwanhäusser Anwaltssozietät
(86) International application number: PCT/JP2004/006910
(87) International publication number: WO 2004/110537

(57) **Abstract**

A mouthpiece 15 is configured to be provided with a plurality of air nozzles 22 which are positioned around an admission port, and from which the air to enclose the surroundings of medicine powder flow discharged from the admission port 20 is blown off. Accordingly, when the breath is inhaled while an outer body 21 of the mouthpiece 15 is being held in the mouth, the air to be blown off from the: air nozzles 22 forms an air curtain 24 to enclose the surroundings of the medicine powder flow 23 discharged from the admission port 20. Thereby, medicine powder included in the medicine powder flow 23 is prevented, by the air curtain 24, from adhering to the surface of the tongue, the internal surface of the oral cavity and the like. Thus, the medicine powder can be inhaled comfortably while unnecessary taste of the medicine powder such as sweetness, saltiness, sourness, bitterness and the like, is not being sensed.

## Description

### FIELD OF THE INVENTION

The present invention relates to an inhalation-type medicine applicator which is preferable in administering powdered medicine (medicine powder), for example, to the lungs or the like of a patient, for example, through the patient's breath inhalation.

### BACKGROUND ART

In general, a method of inhaling powdered medicine (hereinafter referred to as "medicine powder"), filled in a medicine powder container, by use of a specialized medicine applicator has been known as a method of administering medicine to the lungs or the like of a patient suffering from asthma.

In addition, the inhalation-type medicine applicator includes a medicine powder container for containing medicine powder therein and a mouthpiece to be held in the mouth when the medicine powder in the medicine powder container is inhaled. The mouthpiece is provided with an admission port, which is open to the end part of the mouthpiece, and from which a medicine powder flow including the medicine powder discharged from the medicine powder container is inhaled. In addition, the length of the mouthpiece is set at a length which causes the end of the mouthpiece to barely pass over the front teeth when the mouthpiece is held in the mouth. In other words, the length of a part with which the mouthpiece is held in the mouth is set, for example, at approximately 15mm to 25mm (see Japanese Patent Laid-open Official Gazette No. Hei.7-313599, for example.).

Furthermore, in a case where medicine powder is going to be inhaled by use of an inhalation-type medicine applicator according to a conventional technology, the medicine powder container is filled with the medicine powder, the mouthpiece is held in the mouth, and the breath is inhaled through the admission port. Thereby, the external air is flown into the medicine powder container, and the medicine powder is mixed into this air flow, thus supplying the medicine powder in the medicine powder container to the admission port. Thereby, the medicine powder is discharged from the admission port. By this, the medicine powder is administered to the lungs or the like.

With regard to the inhalation-type medicine applicator according to the conventional technology, however, the medicine powder to be discharged along with the air from an input port is discharged to spread in the oral cavity. This causes parts of the medicine powder to adhere to the surface of the tongue, the internal surface of the oral cavity and the like. In this occasion, the tongue senses bitterness and the like due to the adhesion of the medicine powder to the tongue. This brings about a problem that the medicine powder can not be inhaled comfortably.

Furthermore, since the length of the mouthpiece is set at a length (for example, 15mm to 25mm) which allows the mouthpiece to barely pass over the front teeth, the medicine powder to be discharge from the admission port is apt to adhere to the tongue. This brings about another problem that the bitterness of the medicine powder is sensed.

### DISCLOSURE OF THE INVENTION

With the problems with the conventional technology into consideration, the present invention has been made. An object of the present invention is to provide an inhalation-type medicine applicator which prevents inhaled medicine powder from adhering to the tongue, the internal surface of the oral cavity and the like, and which accordingly relieves discomfort due to the taste sensation, thereby enabling the medicine powder to be inhaled comfortably.

According to the present invention, the mouthpiece is provided with air nozzles through which the air is blown off to an admission port through which a medicine powder flow is inhaled. This enables the medicine powder flow to be discharged from the admission port, and enables the air to be blown off from the air nozzles to the admission port when the breath is inhaled while the mouthpiece is being held in the mouth. This makes it possible to blow off the air around the medicine powder flow. Accordingly, the air to be blown off from the air nozzles inhibits the medicine powder from spreading in the oral cavity, thus preventing the medicine powder from adhering to the surface of the tongue, the internal surface of the oral cavity, and the like. This enables a patient to inhale the medicine powder comfortably without sensing unnecessary taste including sweetness, saltiness, sourness, bitterness and the like.

In addition, since the air nozzles are designed to be open to a position deeper inside beyond the end part of the mouthpiece, the air to be blown off from the air nozzles can be put into, order in the mouthpiece before the air reaches the end part of the mouthpiece. Thus, the air curtain can be formed in a stable manner.

Furthermore, since the aforementioned air nozzles are designed to be open to the end part of the mouthpiece, the air which blows off the medicine powder flow from the air nozzles can control a direction in which the medicine powder flows reliably.

Moreover, since the aforementioned air nozzles are set to blow off the air which encloses the surroundings of the medicine powder flow to be discharged from the admission port, the air which behaves like a curtain can inhibit the medicine powder from spreading in the oral cavity. This can prevent the medicine powder from adhering to the surface of the tongue, the internal surface of the oral cavity and the like. This makes it possible for a patient to inhale the medicine powder comfortably without sensing unnecessary taste of the medicine powder, for example, sweetness, saltiness, sourness, bitterness and the like of the medicine powder.

Additionally, since the length of a part of the mouthpiece which is held in the mouth is set in a range of 30mm to 80mm, the end of the mouthpiece can be inserted deep into the oral cavity. This enables the medicine powder to be discharged in a position, for example, beyond gustatory organs in the tongue. Thereby, a patient can inhale the medicine powder comfortably without sensing sweetness, saltiness, sourness, bitterness and the like of the medicine powder when the patient is going to inhale the medicine powder. In addition, if the length of the part of the mouthpiece which is held in the mouth were set at such a length, the mouthpiece can press down the tongue which is an obstacle against the administering of the medicine powder, thus enabling the medicine powder to be discharged directly to the respiratory tract. Accordingly, the medical powder can be prevented from adhering to the tongue, the internal surface of the oral cavity and the like.

### BRIEF DESCRIPTION OF THE DRAWINGS

Fig. 1 is a front view showing an inhalation-type medicine applicator according to a first embodiment of the present invention.
Fig. 2 is a longitudinal sectional view showing the inhalation-type medicine applicator according to the first embodiment of the present invention.
Fig. 3 is a right side view of the inhalation-type medicine applicator shown in Fig. 1.
Fig. 4 is a right side view of the medicine applicator main body from which a capsule holder is detached.
Fig. 5 is a magnified, longitudinal sectional view showing the capsule holder in a magnified manner.
Fig. 6 is a transverse cross-section showing eccentric paths and the like, in a magnified manner, from the VI-VI direction indicated by arrows in Fig. 2.
Fig. 7 is a transverse cross-section showing an inner body and an outer body, in a magnified manner, from the VII-VII direction indicated by arrows in Fig. 2.
Fig. 8 is a transverse cross-section showing the inner body and the outer body, in a magnified manner, from the VIII-VIII direction indicated by arrows in Fig. 2.
Fig. 9 is a longitudinal sectional view showing the inhalation-type medicine applicator in a condition of inhaling the air from an admission port.
Fig. 10 is a longitudinal sectional view showing an inhalation-type medicine applicator according to a second embodiment of the present invention.

### DETAILED DESCRIPTION OF THE PREFERRED EMBODIMENTS

Hereinbelow, descriptions will be provided for an embodiment of the present invention on the basis of the drawings.

A gist of the present invention is that the inhalation-type medicine applicator according to the present invention is an inhalation-type medicine applicator including a medicine powder container for containing medicine powder therein, and a mouthpiece, and that the mouthpiece is provided with air nozzles from which to blow off the air to an admission port of the mouthpiece. Incidentally, configurations of the air nozzles include one in which the air nozzles are open to a position deep inside from the end part of the mouthpiece, and another in which the air nozzles are open to the end part of the mouthpiece. In addition, it is preferable that these air nozzles be formed to blow off the air which encloses the surroundings of a medicine powder flow to be discharged from the admission port of the mouthpiece.

Hereinbelow, detailed descriptions will be provided for inhalation-type medicine applicators respectively according to the embodiments of the present invention, giving an example of the administering of medicine powder to a patient suffering, for example, from bronchial asthma, with reference to the attached drawings.

### (First Embodiment)

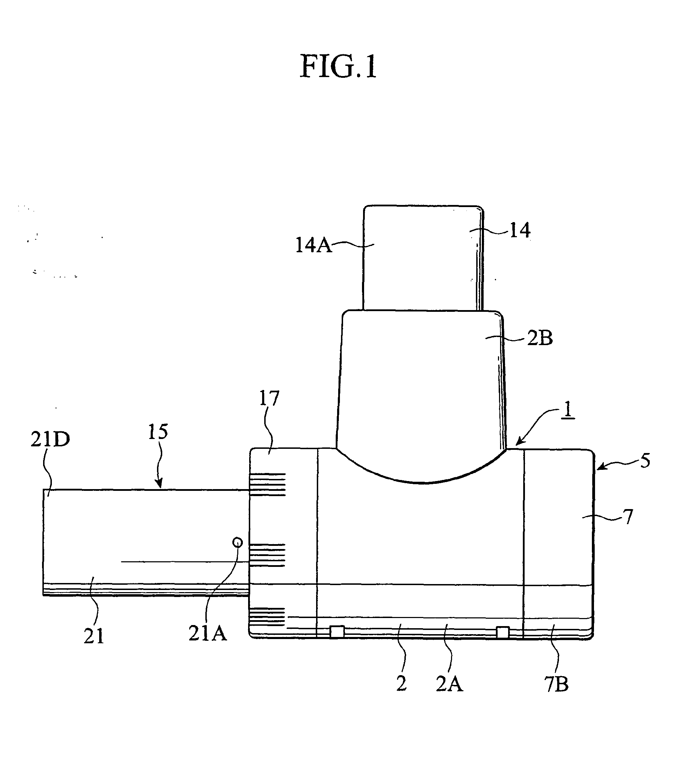

First of all, Figs. 1 to 9 respectively show a first embodiment of the present invention. In Fig. 1, reference numeral 1 denotes a medicine applicator main body which constitutes the base of the inhalation-type medicine applicator. This medicine applicator main body 1 is constituted chiefly of a body 2, a capsule holder 5, a capsule container 8 as a medicine powder container, and the like, all of which will be described later.

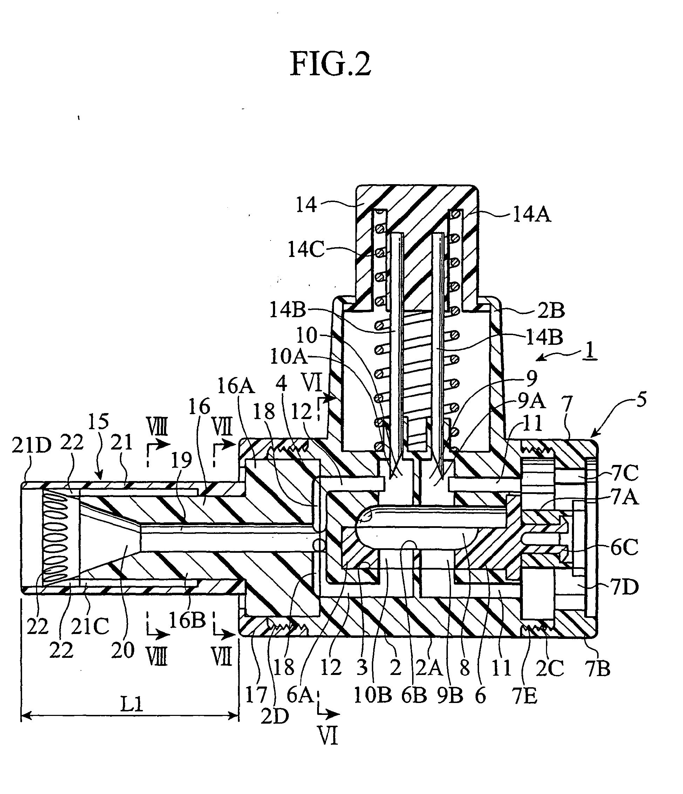

The body 2 is positioned on one side of the medicine applicator main body 1, and constitutes an external shape of the medicine applicator main body 1. As shown in Figs. 1 and 2, this body 2 is constituted of: a cylinder-shaped body 2A formed virtually in a cylinder; and a hole-maker guide 2B, shaped like a long cylinder, which is installed to protrude upward from the outer periphery of the cylinder-shaped body 2A, and which movably supports a supporting part 14A of a hole-maker 14, which will be described later. In addition, a screw thread of a screw part 2C to which a below-described cap 7 is screwed is formed on one side of the cylinder-shaped body 2A. A screw thread of another screw part 2D, to which a screw cylinder 17 of a below-described mouthpiece 15 is screwed, is formed on the other side of the cylinder-shaped body 2A. Furthermore, a holder containing part 3, which will be described later, is formed in the inner periphery of the cylinder-shaped body 2A.

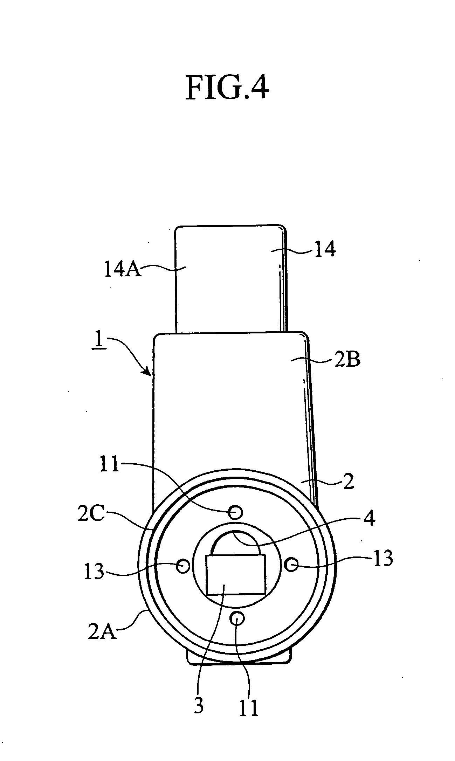

The holder containing part 3 is formed in the inner periphery of the cylinder-shaped body 2A of the body 2. The holder containing part 3 contains a capsule holding part 6 of a capsule holder 5, which will be described later, in a way that the capsule holding part 6 may come out of, and into, the holder containing part 3. As shown in Fig. 4, the holder containing part 3 is positioned in the lower portion of the cylinder-shaped body 2A and in the middle of the cylinder-shaped body 2A in the axis direction thereof. The holder containing part 3 is formed as a closed-end hole, shaped like a quadrangle, which extends in the same axis direction as the cylinder-shaped body 2A does, and which opens on one end thereof.

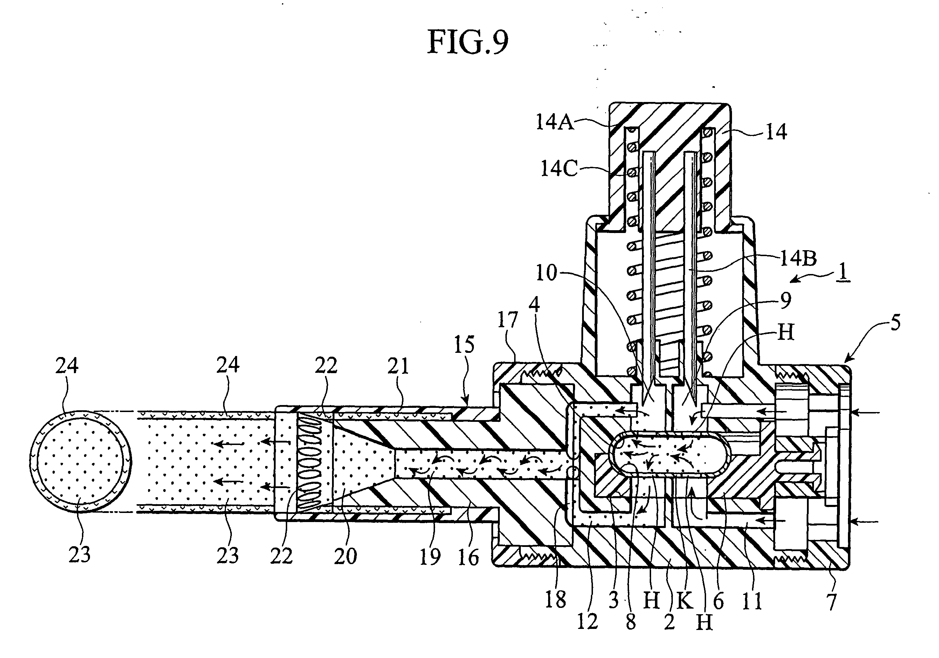

A capsule fitting groove 4 is formed in the upper surface inside the holder containing part 3. Along with a capsule fitting concave part 6B of the capsule holding part 6 which will be described later, this capsule fitting groove 4 constitutes a capsule container 8. In addition, the capsule fitting groove 4 holds a capsule K (see Fig. 9) from the above, and is formed as a groove whose cross section is shaped like a half arc corresponding to the outer diameter of the capsule K.

The capsule holder 5 is provided detachably to the body 2. Along with the aforementioned body 2 and the like, this capsule holder 5 constitutes the medicine applicator main body 1. In addition, the capsule holder 5 is constituted chiefly of the capsule holding part 6 and the cap 7, both of which will be described later.

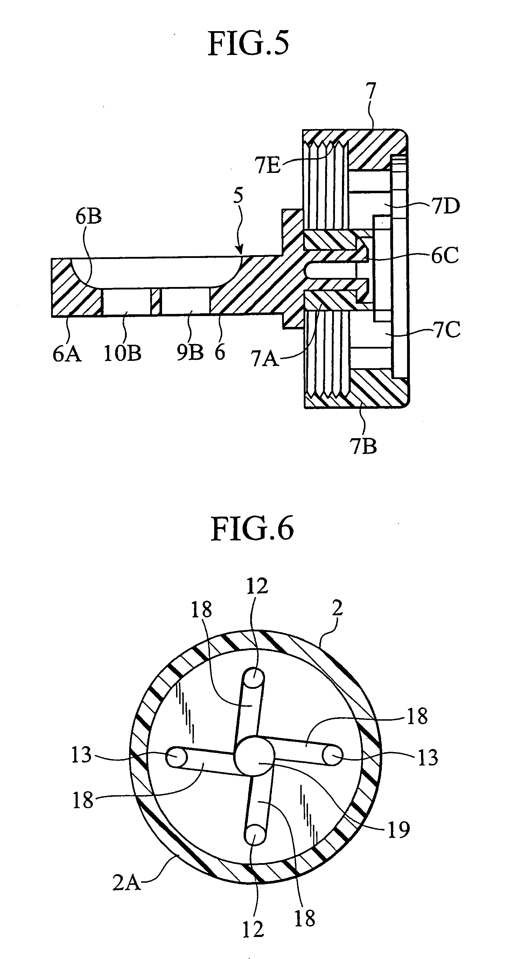

The capsule holding part 6 constitutes the main body of the capsule holder 5, and is provided to the holder containing part 3 in a way that the capsule holding par 6 can come out of, and into, the holder containing part 3. Specifically, as shown in Fig. 5, the capsule holding part 6 is constituted chiefly of: a drawing part 6A formed to extend in the axis direction within the holder containing part 3 ; the capsule fitting concave part 6B formed to be long in the axis direction thereof, and formed, as a groove whose cross section is shaped like a half arc, in the upper surface of the drawing part 6A; and an engagement shaft part 6C which protrudes from one end of the drawing part 6A in the axis direction thereof, and which rotatably engages with an inner cylinder part 7A of the cap 7 in a condition where the engagement shaft part 6C is kept from exiting from the inner cylinder part 7A.

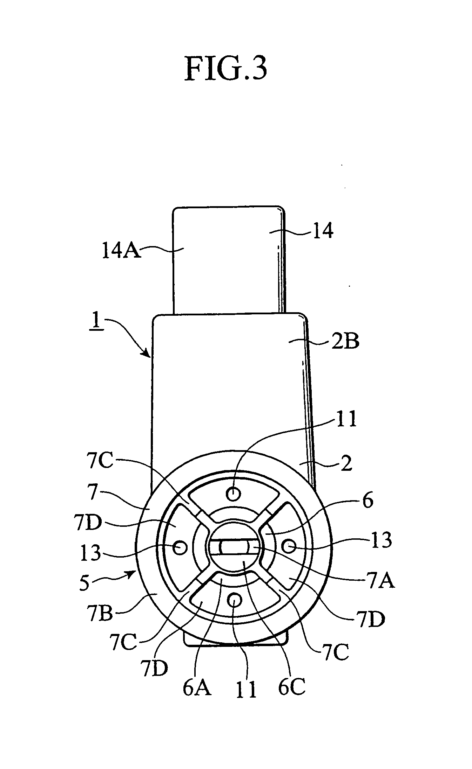

Furthermore, the cap 7 is attached to one end of the capsule holding part 6. As shown in Figs. 3 and 5, this cap 7 is constituted chiefly of: the inner cylinder part 7A with a smaller diameter, which is positioned in the center of the cap 7, and to which the engagement shaft part 6C of the capsule holding part 6 is inserted and attached; an outer cylinder part 7B with a larger diameter, which is positioned in the outer periphery of the cap 7; a plurality, for example, four of legs 7C formed in a radial manner in order to connect the outer cylinder part 7B to the inner cylinder part 7A; air intakes 7D formed between each neighboring two of the legs 7C; and an screw part 7E whose screw thread is formed in the inner periphery of the outer cylinder part 7B, and which is screwed to the screw part 2C of the body 2. At this point, each of the air intakes 7D communicates with the respective inflow-side paths 11 and with the respective auxiliary air paths 13, thereby taking in the external air.

As shown in Figs. 2 and 9, the capsule container 8 is formed between the capsule fitting groove 4 and the capsule fitting concave 6B when the capsule holding part 6 is pressed into the holder containing part 3.

This capsule container 8 contains, and holds, the capsule K which is filled with powdered medicine (medicine powder) therein.

The capsule holder 5 thus formed unlocks the cap 7 when the capsule holder 5 is turned, for example, counterclockwise while the cap 7 is being held tightly. In this occasion, only the cap 7 can be turned while the capsule holding part 6 is being. unturned, since the cap 7 and the capsule holding part 6 rotatably engage with each other. Subsequently, when the cap. 7 is unlocked, if the capsule holding part 6 were drawn out, the capsule holding part 6 can be arranged in a drawn position. In this drawn position, the capsule K which is filled with medicine powder can be fitted to the capsule fitting concave part 6B formed in the drawing part 6A, and a capsule which has been used can be removed from the capsule fitting concave part 6B.

In addition, if the drawing part 6A were pressed into the holder containing part 3 and the cap 7 were turned clockwise to be locked while the capsule K is being fitted into the capsule fitting concave part 6B, the capsule holding part 6 can be arranged in the push-in position, and the capsule K can be held in the capsule container 8, as shown in Fig. 9.

Next, in Fig. 2, reference numeral 9 denotes a pin inserting hole on the inflow side, which is provided to be positioned on one side of the capsule container 8. The pin inserting hole 9 is constituted of: a body-side insertion hole 9A formed in the body 2 in a way that the body-side insertion hole 9A penetrates the capsule container 8 in the diameter direction thereof; and a holder-side insertion hole 9B formed in the capsule holder 5 corresponding to the body-side insertion hole 9A.

Furthermore, a pin insertion hole 10 on the outflow side is formed in a position on the other side of the capsule container 8. This pin inserting hole 10 is constituted of a body-side insertion hole 10A and a holder-side insertion hole 10B, both of which penetrate the capsule container 8 in parallel with the pin inserting hole 9 on the inflow side.

A pair of inflow-side paths 11 are provided to the body 2. These inflow-side paths 11 are designed to flow the air into the capsule K in the capsule container 8. In addition, with regard to each of the inflow-side paths 11, one end of it communicates with the external air through the air intake 7D of the cap 7, and the other end of it communicates with the pin inserting hole 9 on the inflow side.

The body 2 is provided with two outflow-side paths 12 through which an air flow including medicine powder is discharged from the capsule K in the capsule container 8. With regard to each of the outflow-side paths 12, one end of it communicates with the pin inserting hole 10 on the outflow side, and the other end of it communicates with one of eccentric paths 18, which will be described later.

In addition, the aforementioned pin inserting hole 9 and the inflow-side paths 11 constitute an air path on the inflow-side which supplies the external air, which is taken in from the air intakes 7D of the capsule holding part 6, to the capsule container 8 (capsule K) . The pin inserting hole 10 and the outflow side paths 12 constitute an air path on the outflow side through which medicine powder filled in the capsule K, along with the air, is discharged to an admission port 20.

As shown in Figs. 3, 4 and 6, reference numeral 13 denotes two auxiliary paths drilled in the cylinder-shaped body 2A in a way that the two auxiliary paths penetrate the cylinder-shaped body 2A of the body 2 in the axis direction thereof in the respective positions shifted respectively from the paths 11 and 12 by 90°. With regard to each of the auxiliary air paths 13, one end of it communicates with the external air, and the other end of it communicates with one of the eccentric paths 18. At this point, the auxiliary air paths 13 have a role in: relieving a choking sensation, which the user of this inhalation-type medicine applicator feels, by increasing an amount of the air flowing when the user inhales his/her breath; and spreading medicine powder through their communicating with the eccentric paths 18.

The hole-maker 14 is provided to the body 2. This hole maker 14 makes holes in the capsule K contained in the capsule container 8. In addition, as shown in Fig. 2, the hole maker 14 is constituted chiefly of: a supporting part 14A supported movably in the hole-maker guide 2B of the body 2; pins 14B and 14B extending to the respective pin inserting holes 9 and 10 from the supporting part 14A; and a return spring 14C provided between the supporting 14A and the cylinder-shaped body 2A.

In addition, the return spring 14C applies a force to the supporting part 14A in the same direction as each of the pins 14B comes apart from the capsule K. The return spring 14C returns the supporting part 14A and each of the pins 14B to their respective initial positions after the holes are made in the capsule K. Furthermore, the tip of each of the pins 14B is shaped like a sharp needle tip having an inclined plane.

In this manner, the hole maker 14 presses the supporting part 14A into the hole-maker guide 2B against the return spring 14C, and inserts the pins 14B and 14B respectively into the pin inserting holes 9 and 10. Thereby, as shown in Fig. 9, the tips respectively of the pins 14B are pierced into the capsule K in the capsule container 8, thus making four holes H which penetrate the capsule K in the diameter direction of thereof.

Then, the mouthpiece 15 is provided to the other end part of the body 2. This mouthpiece 15 is held in the mouth when medicine powder in the capsule K is inhaled. In addition, the mouthpiece 15 is constituted chiefly of an inner body 16, a screw cylinder 17, the admission port 20, an outer body 21, air nozzles 22 and the like, all of which will be described later.

The inner body 16 constitutes the main body of the mouthpiece 15. This inner body 16 is formed as a stepped circular cylinder having a larger-diameter part 16A fitted to the other end part of the body 2 and a smaller-diameter part 16B protruding from the larger diameter part 16A towards one side in the same axis direction as the larger-diameter part 16A does. In addition, the inner body 16 is installed into the body 2 by screwing the screw cylinder 17, which engages with the outer periphery of the larger-diameter part 16A, into the screw part 2D of the body 2.

Moreover, as shown in Fig. 6 , reference numeral 18 denotes a plurality, for example four of eccentric paths provided to extend across the body 2 and the inner body 16. As shown in Fig. 6, each of the eccentric paths 18 is provided to extend in a radial manner. In addition, with regard to two of the four eccentric paths 18, one ends respectively of them communicate with outflow-side paths 12. With regard to the remaining two eccentric paths, one ends respectively of them communicate with the auxiliary air paths 13. Furthermore, the other end of each of the eccentric paths 18 opens to a diffusion path 19 in a position eccentric in the tangent direction of the diffusion path 19. Thereby, the eccentric paths 18 can turn an air flow, which enters the diffusion path 19, into a revolving flow along the inner surface of the diffusion path 19.

The diffusion path 19 is provided to extend in the same axis direction as the inner body 16 does with the axis of the inner body 16 defined as the center of the cross section of the diffusion path 19. When the revolving flow is formed by each of the eccentric paths 18, this diffusion path 19 causes the revolving flow to diffuse, and atomize, medicine powder.

The admission port 20 is provided to open to the other side of the inner body 16. This admission port 20 is arranged in virtually the same axis line as the aforementioned diffusion path 19 is. In addition, the medicine powder which is diffused and atomized in the diffusion path 19 is discharged (given off) from this admission port 20. The admission port 20 is formed to open gradually larger from the diffusion path 19 towards the side opposite to the diffusion path 19.

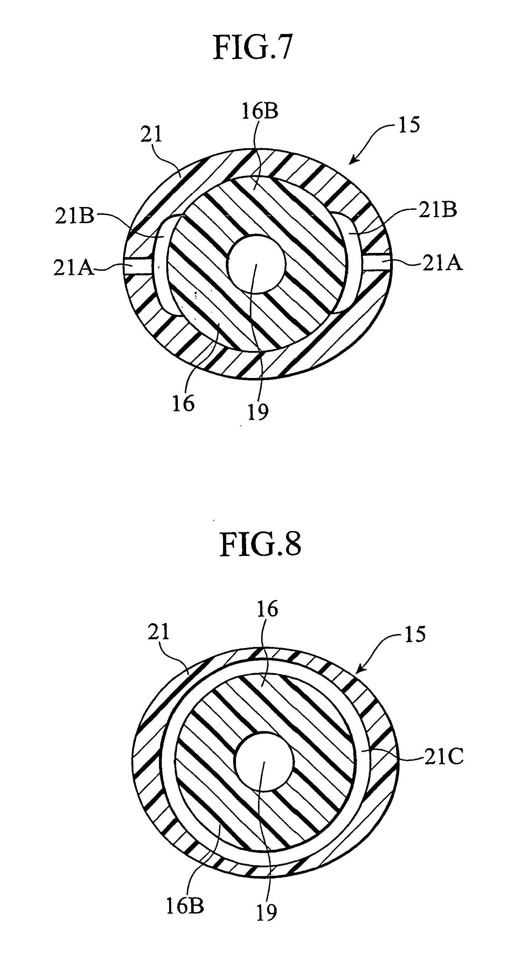

The outer body 21 is provided to the outer periphery of the smaller-diameter part 16B of the inner body 16, and is shaped like a cylinder. The outer body 21 constitutes a held-in-the-mouth part which a patient holds in his/her mouth. The transverse cross-sectional shape of the outer body 21 is shaped like an ellipse long in the horizontal direction which makes it easy for the patient to hold the outer body 21 in his/her mouth, as shown in Figs. 7 and 8. In addition, the outer body 21 is integrally installed to the smaller-diameter part 16B of the inner body 16 by means of adhesion, welding, concave/convex fitting or the like.

At this point, the outer body 21 is provided with two air intake holes 21A which are positioned closer to the larger-diameter part 16A, as shown in Figs. 1 and 7. An arc path 21B whose transverse cross section is shaped like an arc is provided to the inner periphery of the outer body 21 corresponding to each of the air intake holes 21A, and between the smaller-diameter part 16B and the outer body 21. Each of the arc paths 21B extends in the axis direction. In addition, as shown in Fig. 8, a cylinder path 21C which communicates with the downstreams (the other side) respectively of the arc paths 21B is provided to the inner periphery of the outer body 21. Furthermore, one end part of the outer body 21 constitutes an excurrent part 21D which extends frontward from the admission port 20. The air nozzles 22, which will be described later, are provided to the inner periphery of the excurrent part 21D..

A plurality of air nozzles 22 are provided to the inner periphery of the excurrent part 21D of the outer body 21. The air nozzles 22 are formed respectively as circular holes which are arranged in a line in the circumferential direction so as to surround the admission port 20. In addition, the air nozzles 22 blow off the air in a condition of being arranged in a line in the circumferential direction, and the air thus blown off forms an air curtain 24 to enclose the surroundings of the medicine powder flow 23 which has been discharged from the admission port 20, as shown in Fig. 9. A function of the air curtain prevents the medicine powder from adhering to the surface of the tongue, the internal surface of the oral cavity and the like.

Furthermore, each of the air nozzles 22 is provided in a position inwards (towards one end) from the end of the outer body 21 which constitutes the end part of the mouthpiece 16. Thereby, the air to be blown off from the air nozzles 22 can be put into order until the air reaches the end part of the outer body 21, and thus the air curtain 24 can be formed in a cylinder stably.

Hereinafter, descriptions will be provided for the anatomy of the mouth. The inside of the mouth constitutes the oral cavity. The distance between the lips and the pharynx which is located the deepest in the oral cavity, and which leads to the esophagus and the bronchi, is measured approximately 80mm. In addition, gustatory organs which sense taste exit in the tongue in the oral cavity. With regard to these gustatory organs, sweetness sensing parts, saltiness sensing parts, sourness sensing parts and bitterness sensing parts (none of which are illustrated) are arranged in this order inwards from the tip of the tongue. Furthermore, the sweetness sensing parts are arranged up to a position inwards approximately 30mm away from the lips, and the bitterness sensing parts are arranged up to a position inwards approximately 60mm away from the lips. Moreover, each groups of the sensing parts has an important role of sensing taste of food and a drink when the food and the drink put into the oral cavity 22 adhere to the group of the sensing parts. However, each of the groups of the sensing parts senses taste of medicine powder, which needs not be sensed, when the medicine powder adheres to each of the groups of the sensing parts. Accordingly, a patient may feel discomfort while inhaling the medicine powder.

With this taken into consideration, the length L1 in the axis direction of the outer body 21 which constitutes the held-in-the-mouth part is set in a range of 30mm to 80 mm, for example, at approximately 30mm. Thereby, when medicine powder is intended to be inhaled, the medicine powder can be discharged in a position beyond the sweetness sensing parts in the end part of the tongue. This enables the medicine powder to be inhaled comfortably.

The inhalation-type medicine applicator according to the first embodiment has the aforementioned configuration. Next, descriptions will be provided for a motion in which a patient inhales medicine powder.

First of all, the capsule holder 5 is drawn out of the holder containing part 3, and the capsule K is fitted to the capsule fitting concave part 6B of the capsule holding part 6. While this state is being kept, the capsule holding part 6 is pressed into the holder containing part 3, and the capsule K is set in the capsule container 8. Subsequently, the supporting part 14A of the hole-maker 14 is pressed in along the hole-maker guide 2B. Thereby, the pins 14B make the respective four holes H in the capsule K.

After each of the holes H is made in the capsule K in the capsule container 8 in this manner, the patient holds the outer body 21 of the mouthpiece 15, and inhales a breath. Thereby, the medicine powder (the medicine powder flow 23) to be discharged from the admission port 20 can be inhaled.

In addition, while the medicine powder is being inhaled, the air is blown off from the admission port 22, and forms an air curtain 24 to enclose the medicine powder flow from its surroundings. For this reason, the medicine powder included in the medicine powder flow 23 can be prevented from adhering to the surface of the tongue, the internal surface of the oral cavity and the like. This enables the patient to inhale a prescribed amount of medicine powder without sensing taste stemming from sweet ingredients, salty ingredients, sour ingredients and bitter ingredient of the medicine powder.

Moreover, since the length L1 in the axis direction of the outer body 21 is set at 30mm, the medicine powder can be discharged in a position beyond the sweetness sensing parts which are arranged in the tip of the tongue. This enables the medicine powder to be inhaled while taste stemming from sweet ingredients is not being sensed.

According to the first embodiment, the mouthpiece 15 is configured to be provided with a plurality of air nozzles 22 which are positioned around the admission port 20, and from which the air to enclose the surroundings of the medicine powder flow 23 discharged from the admission port20 is blown off. Accordingly, when the breath is inhaled while the outer body 21 of the mouthpiece 15 is being held in the mouth, the air to be blown off from the air nozzles 22 can form an air curtain to enclose the surroundings of the medicine powder flow 23 discharged from the admission port 20.

As a result, the medicine powder included in the medicine powder flow 23 can be prevented, by the air curtain 24, from adhering to the surface of the tongue, the internal surface of the oral cavity and the like. Thereby, the patient can inhale the medicine powder comfortably without sensing unnecessary taste such as sweetness, saltiness, sourness, bitterness and the like.

Additionally, since the air nozzles 22 are designed to be open to a position inwards from the end part of the mouthpiece 15, the air nozzles 22 can put, into order, the air blown off from the air nozzle 22 until the air reaches the end part of the mouthpiece 15. Accordingly, the air curtain 24 can be formed stably.

In addition, since the length L1 of the outer body 21 constitutes the part which is held in the mouth is set at approximately 30mm, the medicine powder can be discharged in a position, for example, beyond the sweetness sensing parts in the tip of the tongue.

### (Second Embodiment)

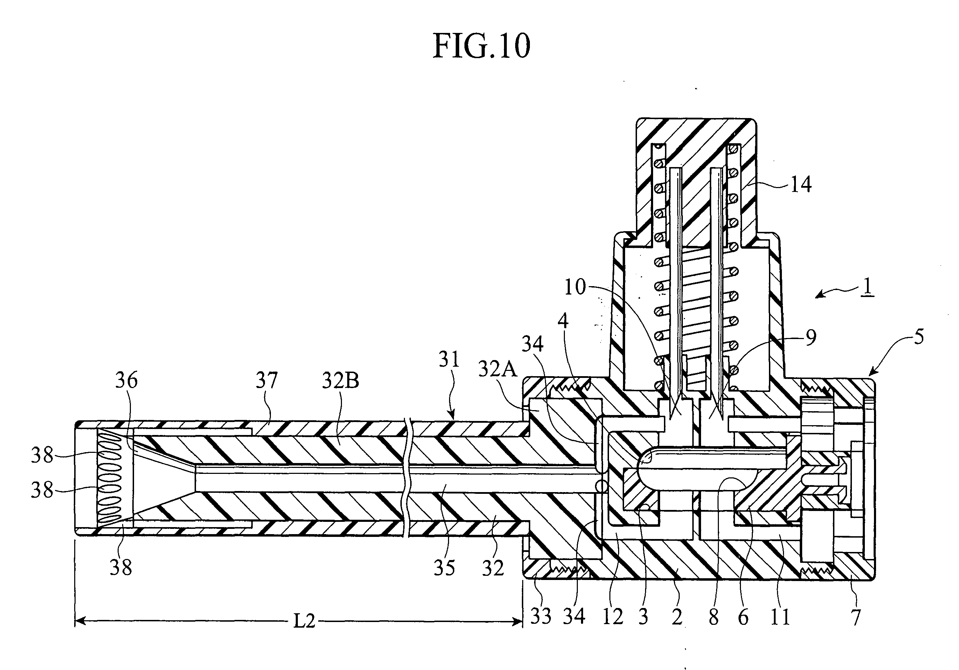

Next, Fig. 10 shows a second embodiment of the present invention. A feature of the second embodiment is that, with regard to the mouthpiece, the length of its part which is held in the mouth is set at approximately 80mm. Incidentally, in the second embodiment, the same reference numerals will be used to denote the same or similar components as those in the first embodiment, so that the descriptions will be omitted.

As shown in Fig. 10, reference numeral 31 denotes a mouthpiece according to the second embodiment which is provided to the other end of the body 2. Virtually like the mouthpiece 15 according to the first embodiment, this mouthpiece 31 is constituted of an inner body 32 formed, as a stepped cylinder body, of a larger-diameter part 32A and a smaller-diameter part 32B; a screw cylinder 33 with which to install the inner body 32 to a body 2; eccentric paths 34 provided between the body 2 and the inner body 32; a diffusion path 35 provided in a position centered on the axis of the inner body 32; an admission port 36; an outer body 37 provided to the outer periphery of the smaller-diameter part 32B of the inner body 32; and air nozzles 38 provided to the outer body 37.

However, the mouthpiece 31 according to the second embodiment is different from the mouthpiece 15 according to the first embodiment in that the outer body 37 which constitutes the held-in-the-mouth part which is held in the mouth is formed to have a larger length, and in that, in conjunction with this, the smaller-diameter part 32B and the like of the inner body 32 are also formed respectively to have longer lengths.

Specifically, with regard to the outer body 37 and the like of the mouthpiece 31, their lengths L2 in the respective axis directions are set at approximately 80mm. If the length L2 of the held-in-the-mouth part were set at approximately 80mm in this manner, when the outer body 37 is inserted deep into the oral cavity, the end of the outer body 37 can be opened to a position beyond the gustatory organs in the tongue sensing sweetness, saltiness, sourness and bitterness. In addition, the outer body, which is 80mm long, can press down the tongue which is an obstacle in the oral cavity, accordingly enabling the medicine powder to be discharged directly to the pharynx.

As described above, the inhalation-type medicine applicator thus configured according to the second embodiment can bring about virtually the same functions and effects as the inhalation-type medicine applicator according to the first embodiment does. In particular, according to the second embodiment, since medicine powder can be discharged directly to the pharynx beyond the gustatory organs in the tongue, the medicine powder can be inhaled comfortably while the taste of the medicine powder is not being sensed, even if the medicine powder includes sweet ingredients, salty ingredients, sour ingredients and bitter ingredients.

In addition, the longer outer body 37 can press down the tongue which is an obstacle, accordingly preventing the medicine powder from adhering to the tongue, the oral cavity and the like, thereby enabling a prescribed amount of medicine powder to be administered to the bronchi and the like efficiently.

### (Other Embodiments)

The descriptions and the drawings which constitute parts of the disclosure of the aforementioned embodiments should not be interpreted as limiting the present invention. Various alternative embodiments, examples and applied techniques may be apparent from this disclosure to those skilled in the art.

Each of the aforementioned embodiments has been described giving the case, for example, of the configuration in which the capsule holder 5 is put out of, and into, the body 2, and thereby the capsule K is loaded and unloaded. However, configurations according to the present invention are not limited to this. For example, as disclosed in Japanese Patent Laid-open Official Gazette No. Hei.7-313599, the inhalation-type medicine applicator may have a configuration in which a capsule is directly put out of, and into, the capsule container.

In addition, according to each of the aforementioned embodiments, the inhalation-type medicine applicator has the configuration in which a capsule K filled with medicine powder is contained in the capsule container 8. However, configurations according to the present invention are not limited to this. For example, the inhalation-type medicine applicator may have a configuration in which the medicine applicator main body is provided with a medicine powder container, accordingly medicine powder is filled directly in the medicine powder container, and thus the medicine powder is inhaled.

Furthermore, the first embodiment has been described giving the example where the plurality of air nozzles 22 are formed as the circular holes arranged in a line in the circumferential direction of the admission port 20 in a way that the air nozzles 22 surround the admission port 20. However, configurations according to the present invention are not limited to this. For example, the inhalation-type medicine applicator may have a configuration in which the air nozzles are formed of arc-shaped slits, or annular slits. This configuration can be applied to the second embodiment.

Moreover, the first embodiment has been described giving the example where the length L1 of the outer body 21 constituting the held-in-the-mouth part of the mouthpiece 15 is set at approximately 30mm. The second embodiment has been described giving the example where the length L2 of the outer body 37 constituting the held-in-the-mouth part of the mouthpiece 31 is set at approximately 80mm. Lengths of the outer body according to the present invention are not limited to these. For example, lengths of the outer body can be set in a range of 30mm to 80mm whenever necessary for each purpose depending on sweet, salty, sour, bitter and other ingredients included in medicine powder.

### INDUSTRIAL APPLICABILITY

According to the present invention, when the breath is inhaled while the mouthpiece is being held in the mouth, the medicine powder flow is discharged from the admission port, and concurrently the air is blown off to the admission port from the air nozzles. Thereby, the air can be blown off to the surroundings of the medicine powder flow. Thus, the air to be blown off from the air nozzles inhibits the medicine powder from spreading in the oral cavity, accordingly preventing the medicine powder from adhering to the surface of the tongue, the internal surface of the oral cavity and the like. By this, a patient can inhale the medicine powder comfortably without sensing unnecessary taste of the medicine powder, such as sweetness, saltiness, sourness, bitterness, and the like.

In addition, since the air nozzles are open to a position inside deeper from the end part of the mouthpiece, the air to be blown off from the air nozzles can be put into order until the air reaches the end part of the mouthpiece. Accordingly, the air curtain can be formed stably.

Furthermore, since the air nozzles are open to the end part of the mouthpiece, the direction of the medicine powder flow can be securely controlled by the air to be blown off from the air nozzles.

Moreover, since the air nozzles are set to blow off the air which is going to enclose the surroundings of the medicine powder flow discharged from the admission port, the air behaving as if it were a curtain can inhibit the medicine powder from spreading in the oral cavity. This prevents the medicine powder from adhering to the surface of the tongue, the internal surface of the oral cavity and the like. Thereby, a patient can inhale the medicine powder comfortably without sensing unnecessary taste of the medicine powder, for example, sweetness, saltiness, sourness, bitterness and the like.

Additionally, since the length of the part in the mouthpiece which is held in the mouth is set in a range of 30mm to 80mm, the end part of the mouthpiece can be inserted deep inside the oral cavity, thereby enabling the medicine powder to be discharged in a position, for example, beyond the gustatory organs in the tongue. Thereby, when the medicine powder is intended to be inhaled, the medicine powder can be inhaled while sweetness, saltiness, sourness, bitterness and the like of the medicine powder is not being sensed. In addition, if the length were set in this manner, the mouthpiece can press down the tongue which is an obstacle, and discharge the medicine powder directly to the respiratory tract, thus enabling the medicine powder to be prevented from adhering to the tongue, the internal surface of the oral cavity and the like.

## Claims

1. An inhalation-type medicine applicator comprising:
a medicine powder container configured to contain medicine powder therein;
a mouthpiece configured to be held in the mouth when the medicine powder in the medicine powder container is inhaled;
and
an admission port, which is provided to the mouthpiece, configured to introduce a medicine powder flow including medicine powder discharged from the medicine powder container, wherein
the mouthpiece is provided with air nozzles from which the air is blown off to the admission port.

2. The inhalation-type medicine applicator according to claim 1, wherein
the air nozzles are open to a position deep inside from an end part of the mouthpiece.

3. The inhalation-type medicine applicator according to claim 1, wherein the air nozzles are open to an end part of the mouthpiece.

4. The inhalation-type medicine applicator according to any one of claim 1, wherein
the air nozzles are configured to blow off air which encloses the surroundings of the medicine powder flow discharged from the admission port.

5. The inhalation-type medicine applicator according to any one of claim 1, wherein
the mouthpiece includes a part thereof which is held in the mouth is set in a range of 30mm to 80mm in length.
